# EUROPEAN PATENT SPECIFICATION

(11) **EP 3 961 642 B1**
(45) Date of publication and mention of the grant of the patent: **22.04.2026**
(21) Application number: 21193200.9
(22) Date of filing: 26.08.2021
(51) Int. Cl.: G16H 20/17, G16H 40/63

(54) **ADJUSTING SETTINGS TO DIFFERENT DEGREES OF AGGRESSIVENESS IN AN AUTOMATED INSULIN DELIVERY SYSTEM BASED ON QUALITY OF BLOOD GLUCOSE LEVEL CONTROL BY A USER**
EINSTELLUNGSANPASSUNG AUF VERSCHIEDENE AGGRESSIVITÄTSGRADE IN EINEM AUTOMATISCHEN INSULINABGABESYSTEM, DAS AUF DER QUALITÄT DER BLUTZUCKERSPIEGEL-KONTROLLE DURCH EINEN BENUTZER BASIERT
AJUSTEMENT DE RÉGLAGES À DIFFÉRENTS DEGRÉS D'AGRESSIVITÉ DANS UN SYSTÈME AUTOMATIQUE D'ADMINISTRATION D'INSULINE, BASÉ SUR LA QUALITÉ DE CONTRÔLE DE LA GLYCÉMIE PAR L'UTILISATEUR

(30) Priority: 31.08.2020 US 202017008101
(43) Date of publication of application: 02.03.2022
(73) Proprietor: Insulet Corporation, Acton, MA 01720 (US)
(72) Inventor: BENJAMIN, Eric, Cambridge (US); O'CONNOR, Jason, Acton (US); ZHENG, Yibin, Hartland (US); LEE, Joon Bok, Acton (US); BOYNS, Mark, Alpine (US)
(74) Representative: Peterreins Schley

(56) References cited:
- WO-A1-2020/172628
- US-A1- 2017 203 038
- US-A1- 2019 336 683
- US-A1- 2020 101 225

## Description

### BACKGROUND

An automated insulin delivery (AID) system provides automated delivery of insulin to a user via a delivery mechanism, like an insulin pump. The delivery mechanism may be secured to the user to help regulate the blood glucose level of the user. The delivery mechanism includes components that physically interface with the user. The components may include, for instance, needles, a cannula and tubes for enabling the insulin to be delivered from the delivery mechanism to the user. The AID system typically relies on blood glucose level readings from a glucose monitor to regulate insulin delivery to the user.

The AID system may employ a closed loop control approach or an open control loop approach. With a closed loop control approach, the AID system aims to keep the blood glucose level of the user at a control target level without user input and adjusts insulin delivery based on how far the blood glucose level of the user is from the control target level. Constraints may be used to help avoid hypoglycemia, hyperglycemia and other undesired outcomes. A cost function may be used to balance the delivery of insulin with costs associated with the delivery (such as hypoglycemic risk). Each cost in the cost function may be assigned a weight.

The AID system is typically configured to have initial settings. These initial settings may include things such as the control target level, constraints (like maximum change in basal dosage per cycle) and weights for the cost function. Conventional AID systems adopt a one size fits all approach to the initial settings. Each user is assigned the same initial settings. Over time the AID systems may adjust those settings, but the adjustment typically is a slow process.

This one size fits all approach is not optimal for many users. For example, many users may be better served by different target levels, tighter constraints or looser constraints and different cost function weights. Instead, with conventional systems, the users must endure the less well-suited settings until the AID system adapts the settings over an extended period of time.

Prior art document US 2017/203038 A1 (DESBOROUGH LANE [US] ET AL) 20 July 2017 (2017-07-20) discloses an AID system wherein setting are adapted based on variability of continuous glucose monitor (CGM) and/or blood glucose monitor (BGM) data.

### SUMMARY

The invention is defined by the method of independent claim 1 and the system of independent claim 12. Optional features of the invention are provided by the dependent claims.

### BRIEF DESCRIPTION OF THE DRAWINGS

Figure 1A depicts a block diagram of a control loop for an AID System.
Figure 1B depicts a flowchart of illustrative steps performed in the control loop of Figure 1A.
Figure 2 depicts a block diagram of an AID system that is suitable for exemplary embodiments.
Figure 3 depicts a flowchart of illustrative steps that may be performed to adjust or consider adjusting settings for the AID system.
Figure 4 depicts a diagram of illustrative types of triggers for triggering whether to change or consider changing the settings of the AID system.
Figure 5 depicts a flowchart of illustrative steps that may be performed to adjust settings of the AID system.
Figure 6 depicts a block diagram of illustrative metrics of glucose control quality.
Figure 7 depicts illustrative types of more aggressive/less aggressive settings.
Figure 8 depicts a flowchart of illustrative steps that may be performed to enable settings based on quality of blood glucose level control of the user.
Figure 9 depicts a flowchart of illustrative steps that may be performed to make the enablement of additional settings a game to encourage good quality blood glucose level control.
Figure 10 depicts a flowchart of illustrative steps that may be performed by a healthcare professional regarding the settings.

### DETAILED DESCRIPTION

The exemplary embodiments allow a user to have more aggressive settings for an AID system after demonstrating good blood glucose level control. This allows the settings to be more quickly customized to users that demonstrate good quality blood glucose level control than conventional systems. As these users have demonstrated good quality glucose level control there is less need to constrain the settings and provide a high margin of safety. Conversely, users demonstrating poor quality blood glucose level control may have less aggressive settings imposed.

Aggressiveness in settings refers to the risk potential posed by the settings. For example, suppose that conventional settings permit a control blood glucose level target to be in the range between 110 mg/dL to 140 mg/dL. More aggressive settings may loosen the constraints to permit the control blood glucose level target to be in the range between 90 mg/dL and 150 mg/dL. The expanded range poses a greater risk because choosing a value in the expanded range, such as 90 mg/dL, may increase the hypoglycemic risk for the user as the user may be more likely to experience a hypoglycemic event with that setting, whereas choosing a control blood glucose level target value, such as 150 mg/dL, in the expanded range may increase the hyperglycemic risk of the user as the user is more likely to experience a hyperglycemic event with that setting. Setting the control values to 90 mg/dL or 150 mg/dL are also examples of more aggressive settings.

Conversely, less aggressive settings are those that pose less potential risk to the user. For example, a user may only need a more limited range of control blood glucose level targets available. In that case, the permissible blood glucose level target range constraint may be tightened to be between 120 mg/dl and 135 mg/dl, and the control blood glucose level target may be set at 125 mg/dL. The less aggressive settings may be set as the initial default settings, or in response to poor quality blood glucose level control. Alternatively, the transition to less aggressive settings may be set as a matter of convenience or ease of use for the user. Other, factors, such as the user's age, health, cognitive state, etc., may weigh in the determination of whether to modify the aggressiveness of the settings.

The exemplary embodiments may observe and analyze the blood glucose level history of a user and determine the quality of blood glucose level control. The determination that the user has managed their blood glucose level control well or poorly may trigger a transition in AID system settings. In some exemplary embodiments, a medical professional may analyze the blood glucose level history and decide to change the aggressiveness of the settings or enable for the user the ability to change the aggressiveness of the settings.

In some exemplary embodiments, the settings may be automatically changed programmatically. In other embodiments, the settings are changed responsive to intervention by a party. In other exemplary embodiments, settings of a different level of aggressiveness become available to a user after exhibiting a quality of blood glucose level control, and the user may choose whether to adopt the settings.

In some exemplary embodiments, the access to new settings is gamified. The user is informed that good quality blood glucose level control will make new settings available. If the user exhibits good quality blood glucose level control, the user is granted access to the settings. In some instances, the user may be granted further access to additional settings, or continued access to the new settings, as good quality blood glucose level control persists. For example, a user may be granted access to increasingly aggressive settings if the user continues to exhibit good quality glucose level control as the user is granted access to more aggressive settings over time. Alternatively, the user may maintain their access to the more aggressive settings if the user maintains good quality glucose level control.

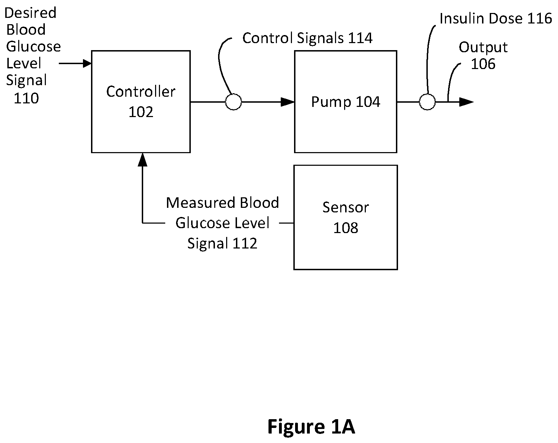

Figure 1A illustrates a simplified block diagram of an example of an AID system 100 suitable for practicing an exemplary embodiment. The example AID system 100 may include a controller 102, a pump mechanism or other fluid extraction mechanism 104 (hereinafter "pump 104"), and a sensor 108. The controller 102, pump 104, and sensor 108 may be communicatively coupled to one another via a wired or wireless communication path. For example, each of the controller 102, the pump 104 and the sensor 108 may be equipped with a wireless radio frequency transceiver operable to communicate via one or more communication protocols, such as Bluetooth^{®}, Bluetooth^{®} Low Energy (BLE), or the like. The sensor 108 may be a glucose monitor, such as, for example, a continuous glucose monitor (CGM) 108. The sensor 108 may, for example, be operable to measure blood glucose (BG) values of a user to generate the measured actual BG level signal 112.

As shown in the example, the controller 102 may receive a desired BG level signal 110, which may be a first signal, indicating a desired BG level or range for a user. The desired BG level signal 110 may be received from a user interface at the controller or other device, or by an algorithm that automatically determines a BG level for a user. The sensor 108 may be coupled to the user and be operable to measure an approximate value of an actual BG level of the user. The measured BG value, the actual BG level, the approximate measured value of the actual BG level are only approximate values of a user's BG level, and it should be understood that there may be errors in the measured BG levels. The errors may, for example, be attributable to a number of factors such as age of the sensor 108, location of the sensor 108 on a body of a user, environmental factors (e.g., altitude, humidity, barometric pressure), or the like. The terms measured BG value, actual BG level, approximate measured value of the actual BG level may be used interchangeably throughout the specification and drawings. In response to the measured BG level or value, the sensor 108 generate a signal indicating the measured BG value. As shown in the example, the controller 102 may also receive from the sensor 108 via a communication path, a measured BG level signal 112, which may be a second signal, indicating an approximate measured value of the actual BG level of the user.

Based on the desired BG level signal 110 and the measured actual BG level signal 112, the controller 102 may generate one or more control signals 114 for directing operation of the pump 104. For example, one of the control signals 114 may cause the pump 104 to deliver an insulin dose 116 to a user via output 106. The insulin dose 116 may, for example, be determined based on a difference between the desired BG level signal 110 and the actual BG signal level 112. A cost function plays a role in determining the dosage as part of the closed loop control system as will be described below. The insulin dose 116 may be determined as an appropriate amount of insulin to drive the actual BG level of the user to the desired BG level. Based on operation of the pump 104 as determined by the control signals 114, the user may receive the insulin 116 from the pump 104.

In various examples, one or more components of the AID system 100 may be incorporated into a wearable or on body drug delivery system that is attached to the user.

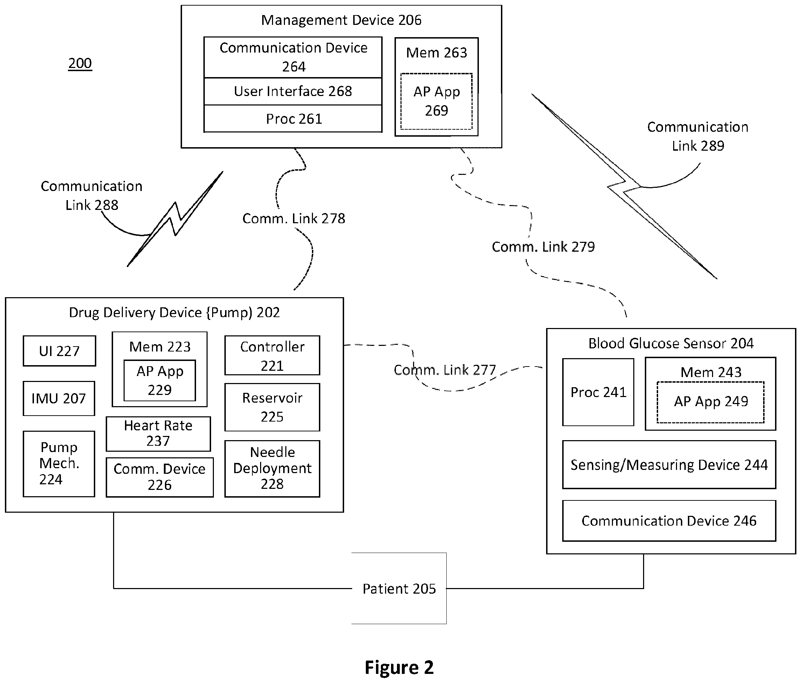

The simplified block diagram of the example AID system 100 provides a general illustration of the operation of the system. An example of a more detailed implementation of devices usable in such an Artificial Pancreas (AP) system is illustrated in Figure 2, which will be described in detail below.

Various examples of an AID system include a wearable drug delivery device that may operate in the system to manage treatment of a diabetic user according to a diabetes treatment plan. The diabetes treatment plan may include a number of parameters related to the delivery of insulin that may be determined and modified by a computer application referred to as an AP application.

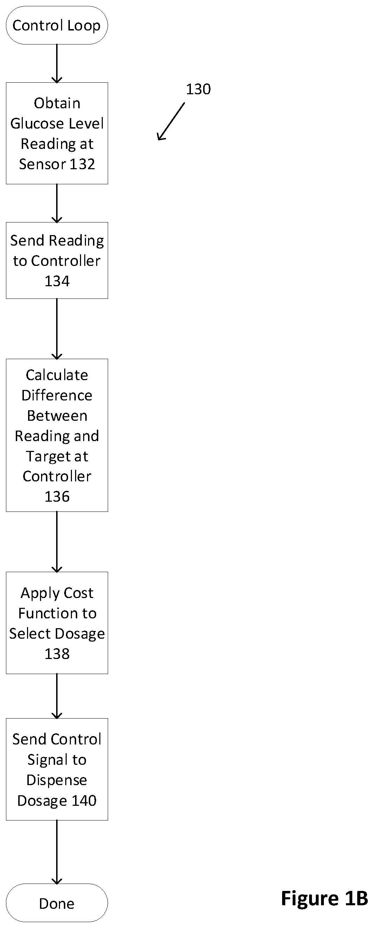

Figure 1B depicts a flowchart 130 of steps that may be performed by exemplary embodiments of the AID system in determining what dose of insulin to deliver to the user as part of the closed loop control system. Initially, as was described above relative to Figure 1A, a BG level reading is obtained by the sensor 108 (132). The BG level reading is sent via a signal 112 to the controller 102 (134). The controller 102 calculates an error value as the difference between the measured BG level 112 and the desired BG level 110 (136). The closed loop control system attempts to minimize the aggregate penalty of the cost function over a wide range of possible dosages. The cost function is applied to the possible dosages, and the dosage with the best penalty function value is selected (138). Depending on how the cost function is configured, the best value may be the lowest value or the highest value. The cost function used in exemplary embodiments will be described in more below. A control signal 114 may be generated by the controller 102 and sent to the pump 104 to cause the pump to deliver the desired insulin dose to the user (140).

Figure 2 illustrates an example of a drug delivery system. The drug delivery system 200 may include a drug delivery device 202, a management device 206, and a BG sensor 204.

In the example of Figure 2, the drug delivery device 202 may be a wearable or on-body drug delivery device that is worn by a user on the body of the user. As shown in Figure 2, the drug delivery device 202 may include an inertial measurement unit (IMU) 207. The drug delivery device 202 may further include a pump mechanism 224 that may, in some examples be referred to as a drug extraction mechanism or component, and a needle deployment mechanism 228. In various examples, the pump mechanism 224 may include a pump or a plunger (not shown).

The needle deployment component 228 may, for example include a needle (not shown), a cannula (not shown), and any other fluid path components for coupling the stored liquid drug in the reservoir 225 to the user. The cannula may form a portion of the fluid path component coupling the user to the reservoir 225. After the needle deployment component 228 has been activated, a fluid path (not shown) to the user is provided, and the pump mechanism 224 may expel the liquid drug from the reservoir 225 to deliver the liquid drug to the user via the fluid path. The fluid path may, for example, include tubing (not shown) coupling the wearable drug delivery device 202 to the user (e.g., tubing coupling the cannula to the reservoir 225).

The wearable drug delivery device 202 may further include a controller 221 and a communications interface device 226. The controller 221 may be implemented in hardware, software, or any combination thereof. The controller 221 may, for example, be a microprocessor, a logic circuit, a field programmable gate array (FPGA), an application specific integrated circuit (ASIC) or a microcontroller coupled to a memory. The controller 221 may maintain a date and time as well as other functions (e.g., calculations or the like) performed by processors. The controller 221 may be operable to provide artificial pancreas (AP) functionality to direct operation of the drug delivery device 202. In addition, the controller 221 may be operable to receive data or information indicative of the activity of the user from the IMU 207, as well as from any other sensors (such as those (e.g., accelerometer, location services application or the like) on the management device 206 or CGM 204) of the drug delivery device 202 or any sensor coupled thereto, such as a global positioning system (GPS)-enabled device or the like.

The controller 221 may process the data from the IMU 207 or any other coupled sensor to determine if an alert or other communication is to be issued to the user and/or a caregiver of the user or if an operational mode of the drug delivery device 202 is to be adjusted. The controller 221 may provide the alert, for example, through the communications interface device 226. The communications interface device 226 may provide a communications link to one or more management devices physically separated from the drug delivery device 202 including, for example, a management device 206 of the user and/or a caregiver of the user (e.g., a parent or HCP). The communication link provided by the communications interface device 226 may include any wired or wireless communication link operating according to any known communications protocol or standard, such as Bluetooth or a cellular standard.

The example of Figure 2 further shows the drug delivery device 202 in relation to a BG sensor 204, which may be, for example, a CGM. The BG sensor 204 may be physically separate from the drug delivery device 202 or may be an integrated component thereof. The BG sensor 204 may provide the controller 221 with data indicative of measured or detected BG levels of the user.

The management device 206 may be maintained and operated by the user or a caregiver of the user. The management device 206 may control operation of the drug delivery device 202 and/or may be used to review data or other information indicative of an operational status of the drug delivery device 202 or a status of the user. The management device 206 may be used to direct operations of the drug delivery device 202. The management device 206 may include a processor 261 and memory devices 263. The memory devices 262 may store an AP application 269 including programming code that may implement the activity mode, the hyperglycemia protection mode, and/or the hypoglycemia protection mode. The management device 206 may receive alerts, notifications, or other communications from the drug delivery device 202 via one or more known wired or wireless communication standards or protocols.

The drug delivery system 200 may be operable to implement the AP application that includes components (e.g., an accelerometer) and functionality to determine a movement of a wearable drug delivery device that is indicative of physical activity of the user, implement an activity mode, a hyperglycemia mode, a hypoglycemia mode, and other functions, such as control of the wearable drug delivery device. The drug delivery system 200 may be an automated drug delivery system that may include a wearable drug delivery device (pump) 202, a BG sensor 204, and a personal diabetes management device (PDM or smartphone) 206.

In an example, the wearable drug delivery device 202 may be attached to the body of a user 205 and may deliver insulin to a user. The wearable drug delivery device 202, for example, may be a wearable device worn by the user. For example, the wearable drug delivery device 202 may be directly coupled to a user (e.g., directly attached to a body part and/or skin of the user via an adhesive or the like). In an example, a surface of the wearable drug delivery device 202 may include an adhesive to facilitate attachment to a user.

The wearable drug delivery device 202 may be referred to as a pump, or an insulin pump, in reference to the operation of expelling a drug from the reservoir 225 for delivery of the drug to the user.

In an example, the wearable drug delivery device 202 may include the reservoir 225 for storing the drug (such as insulin), a needle or cannula (not shown) for delivering the insulin into the body of the user (which may be done subcutaneously, intraperitoneally, or intravenously), and a pump mechanism (mech.) 224, or other drive mechanism, for transferring the drug from the reservoir 225, through a needle or cannula (not shown), and into the user. The reservoir 225 may be configured to store or hold a liquid or fluid, such as insulin. The pump mechanism 224 may be fluidly coupled to reservoir 225, and communicatively coupled to the processor 221. The wearable drug delivery device 202 may also include a power source (not shown), such as a battery, a piezoelectric device, or the like, for supplying electrical power to the pump mechanism 224 and/or other components (such as the processor 221, memory 223, and the communication device 226) of the wearable drug delivery device 202. Although also not shown, an electrical power supply for supplying electrical power may similarly be included in each of the BG sensor 204, the smart accessory device 207 and the management device (PDM) 206.

In an example, the BG sensor 204 may be a device communicatively coupled to the processor 261 or 221 and may be operable to measure a BG value at a predetermined time interval, such as every 5 minutes, or the like. The BG sensor 204 may provide a number of BG measurement values to the AP applications operating on the respective devices. For example, the BG sensor 204 may be a continuous BG sensor that provides BG measurement values to the AP applications operating on the respective devices periodically, such as approximately every 5, 10, 12 minutes, or the like.

The wearable drug delivery device 202 may also include the IMU 207. The IMU 207 may be operable to detect various motion parameters (e.g., acceleration, deceleration, speed, orientation, such as roll, pitch, yaw, compass direction, or the like) that may be indicative of the activity of the user. For example, the IMU 207 may output signals in response to detecting motion of the wearable drug delivery device 202 that is indicative of a status of any physical condition of the user, such as, for example, a motion or position of the user. Based on the detected activity of the user, the drug delivery device 202 may adjust operation related to drug delivery, for example, by implementing an activity mode as discussed herein.

The wearable drug delivery device 202 may, when operating in a normal mode of operation, provide insulin stored in reservoir 225 to the user based on information (e.g., blood glucose measurement values, inputs from an inertial measurement unit, global positioning system-enabled devices, Wi-Fi-enabled devices, or the like) provided by the BG sensor 204 and/or the management device (PDM) 206.

For example, the wearable drug delivery device 202 may contain analog and/or digital circuitry that may be implemented as a controller 221 (or processor) for controlling the delivery of the drug or therapeutic agent. The circuitry used to implement the processor 221 may include discrete, specialized logic and/or components, an application-specific integrated circuit, a microcontroller or processor that executes software instructions, firmware, programming instructions or programming code (enabling, for example, the AP App 229 stored in memory 223, or any combination thereof. For example, the processor 221 may execute a control algorithm, such as an AP application 229, and other programming code that may make the processor 221 operable to cause the pump to deliver doses of the drug or therapeutic agent to a user at predetermined intervals or as needed to bring BG measurement values to a target BG value. The size and/or timing of the doses may be programmed, for example, into an AP application 229 by the user or by a third party (such as a health care provider, wearable drug delivery device manufacturer, or the like) using a wired or wireless link, such as 220, between the wearable drug delivery device 202 and a management device 206 or other device, such as a computing device at a healthcare provider facility. In an example, the pump or wearable drug delivery device 202 is communicatively coupled to the processor 261 of the management device via the wireless link 220 or via a wireless link, such as 291 from smart accessory device 207 or 208 from the BG sensor 204. The pump mechanism 224 of the wearable drug delivery device may be operable to receive an actuation signal from the processor 261, and in response to receiving the actuation signal and expel insulin from the reservoir 225 and the like.

The devices in the system 200, such as management device 206, smart accessory device 207 and BG sensor 204, may also be operable to perform various functions including controlling the wearable drug delivery device 202. For example, the management device 206 may include a communication device 264, a processor 261, and a management device memory 263. The management device memory 263 may store an instance of the AP application 269 that includes programming code, that when executed by the processor 261 provides the process examples described herein. The management device memory 263 may also store programming code for providing the process examples described with reference to the examples herein.

Although not shown, the system 200 may include a smart accessory device that may be, for example, an Apple Watch^{®}, other wearable smart device, including eyeglasses, provided by other manufacturers, a global positioning system-enabled wearable, a wearable fitness device, smart clothing, or the like. Similar to the management device 206, the smart accessory device (not shown) may also be operable to perform various functions including controlling the wearable drug delivery device 202. For example, the smart accessory device may include a communication device, a processor, and a memory. The memory may store an instance of the AP application that includes programming code for providing the process examples described with reference to the examples described herein. The memory may also store programming code and be operable to store data related to the AP application.

The BG sensor 204 of system 200 may be a CGM as described above, that may include a processor 241, a memory 243, a sensing or measuring device 244, and a communication device 246. The memory 243 may store an instance of an AP application 249 as well as other programming code and be operable to store data related to the AP application 249. The AP application 249 may also include programming code for providing the process examples described with reference to the examples described herein.

Instructions for determining the delivery of the insulin (e.g., as a bolus dosage) to the user (e.g., the size and/or timing of any doses of the drug or therapeutic agent) may originate locally by the wearable drug delivery device 202 or may originate remotely and be provided to the wearable drug delivery device 202. In an example of a local determination of insulin delivery, programming instructions, such as an instance of the AP application 229, stored in the memory 223 that is coupled to the wearable drug delivery device 202 may be used to make determinations by the wearable drug delivery device 202. In addition, the wearable drug delivery device 202 may be operable to communicate via the communication device 226 and communication link 288 with the wearable drug delivery device 202 and with the BG sensor 204 via the communication device 226 and communication link 289.

Alternatively, the remote instructions may be provided to the wearable drug delivery device 202 over a wired or wireless link by the management device (PDM) 206. The PDM 206 may be equipped with a processor 261 that may execute an instance of the AP application 269, if present in the memory 263. The memory may store computer-readable instructions for execution by the processor 261. The memory may include a non-transitory computer-readable storage media for storing instructions executable by the processor. The wearable drug delivery device 202 may execute any received instructions (originating internally or from the management device 206) for the delivery of insulin to the user. In this way, the delivery of the insulin to a user may be automated.

In various examples, the wearable drug delivery device 202 may communicate via a wireless communication link 288 with the management device 206. The management device 206 may be an electronic device such as, for example, a smart phone, a tablet, a dedicated diabetes therapy management device, or the like. Alternatively, the management device 206 may be a wearable wireless accessory device, such as a smart watch, or the like. The wireless links 287-289 may be any type of wireless link provided by any known wireless standard. As an example, the wireless links 287-289 may enable communications between the wearable drug delivery device 202, the management device 206 and BG sensor 204 based on, for example, Bluetooth^{®}, Wi-Fi^{®}, a near-field communication standard, a cellular standard, or any other wireless optical or radio-frequency protocol.

The BG sensor 204 may also be coupled to the user by, for example, adhesive or the like and may provide information or data on one or more medical conditions and/or physical attributes of the user. The information or data provided by the BG sensor 204 may be used to adjust drug delivery operations of the wearable drug delivery device 202. For example, the BG sensor 204 may be a glucose sensor operable to measure BG and output a BG value or data that is representative of a BG value. For example, the BG sensor 204 may be a glucose monitor that provides periodic BG measurements a CGM, or another type of device or sensor that provides BG measurements.

The BG sensor 204 may include a processor 241, a memory 243, a sensing/measuring device 244, and communication device 246. The communication device 246 of BG sensor 204 may include an electronic transmitter, receiver, and/or transceiver for communicating with the management device 206 over a wireless link 222 or with wearable drug delivery device 202 over the link 208. The sensing/measuring device 244 may include one or more sensing elements, such as a BG measurement element, a heart rate monitor, a blood oxygen sensor element, or the like. The processor 241 may include discrete, specialized logic and/or components, an application-specific integrated circuit, a microcontroller or processor that executes software instructions, firmware, programming instructions stored in memory (such as memory 243), or any combination thereof. For example, the memory 243 may store an instance of an AP application 249 that is executable by the processor 241.

Although the BG sensor 204 is depicted as separate from the wearable drug delivery device 202, in various examples, the BG sensor 204 and wearable drug delivery device 202 may be incorporated into the same unit. That is, in one or more examples, the BG sensor 204 may be a part of the wearable drug delivery device 202 and contained within the same housing of the wearable drug delivery device 202 (e.g., the BG sensor 204 may be positioned within or embedded within the wearable drug delivery device 202). Glucose monitoring data (e.g., measured BG values) determined by the BG sensor 204 may be provided to the wearable drug delivery device 202, smart accessory device 207 and/or the management device 206, which may use the measured BG values to determine movement of the wearable drug delivery device indicative of physical activity of the user, an activity mode, a hypoglycemia mode and a hyperglycemia mode.

In an example, the management device 206 may be a personal diabetes manager. The management device 206 may be used to program or adjust operation of the wearable drug delivery device 202 and/or the BG sensor 204. The management device 206 may be any portable electronic device including, for example, a dedicated controller, such as processor 261, a smartphone, or a tablet. In an example, the management device (PDM) 206 may include a processor 261, a management device management device memory 263, and a communication device 264. The management device 206 may contain analog and/or digital circuitry that may be implemented as a processor 261 (or controller) for executing processes to manage a user's BG levels and for controlling the delivery of the drug or therapeutic agent to the user. The processor 261 may also be operable to execute programming code stored in the management device management device memory 263. For example, the management device management device memory 263 may be operable to store AP application 269 that may be executed by the processor 261. The processor 261 may, when executing the AP application 269, be operable to perform various functions, such as those described with respect to the examples. The communication device 264 may be a receiver, a transmitter, or a transceiver that operates according to one or more radio-frequency protocols. For example, the communication device 264 may include a cellular transceiver and a Bluetooth transceiver that enables the management device 206 to communicate with a data network via the cellular transceiver and with the BG sensor 204 and the wearable drug delivery device 202. The respective transceivers of communication device 264 may be operable to transmit signals containing information useable by or generated by the AP application or the like. The communication devices 226 and 246 of respective wearable drug delivery device 202 and BG sensor 204, respectively, may also be operable to transmit signals containing information useable by or generated by the AP application or the like.

The wearable drug delivery device 202 may communicate with the BG sensor 204 over a wireless link 208 and may communicate with the management device 206 over a wireless link 220. The BG sensor 204 and the management device 206 may communicate over a wireless link 222. The smart accessory device 207, when present, may communicate with the wearable drug delivery device 202, the BG sensor 204 and the management device 206 over wireless links 287, 288 and 289, respectively. The wireless links 287, 288 and 289 may be any type of wireless link operating using known wireless standards or proprietary standards. As an example, the wireless links 287, 288 and 289 may provide communication links based on Bluetooth^{®}, Wi-Fi, a near-field communication standard, a cellular standard, or any other wireless protocol via the respective communication devices 226, 246 and 264. In some examples, the wearable drug delivery device 202 and/or the management device 206 may include a user interface 227 and 268, respectively, such as a keypad, a touchscreen display, levers, buttons, a microphone, a speaker, a display, or the like, that is operable to allow a user to enter information and allow the management device to output information for presentation to the user.

In various examples, the drug delivery system 200 may be an insulin drug delivery system. For example, the wearable drug delivery device 202 may be the OmniPod^{®} (Insulet Corporation, Acton, MA) insulin delivery device as described in U.S. Pat. No. 7,303,549, U.S. Pat. No. 7,137,964, or U.S. Pat. No. 6,740,059.

In the examples, the drug delivery system 200 may implement the AP algorithm (and/or provide AP functionality) to govern or control automated delivery of insulin to a user (e.g., to maintain euglycemia - a normal level of glucose in the blood). The AP application may be implemented by the wearable drug delivery device 202 and/or the BG sensor 204. The AP application may be used to determine the times and dosages of insulin delivery. In various examples, the AP application may determine the times and dosages for delivery based on information known about the user, such as the user's sex, age, weight, or height, and/or on information gathered about a physical attribute or condition of the user (e.g., from the BG sensor 204). For example, the AP application may determine an appropriate delivery of insulin based on glucose level monitoring of the user through the BG sensor 204. The AP application may also allow the user to adjust insulin delivery, or the AP application may adjust the insulin delivery automatically. For example, the AP application may allow a user to select (e.g., via an input) commands for output to the wearable drug delivery device 202, such as a command to set a mode of the wearable drug delivery device, such as an activity mode, a hyperglycemia protect mode, a hypoglycemia protect mode, deliver an insulin bolus, or the like. In one or more examples, different functions of the AP application may be distributed among two or more of the management device 206, the wearable drug delivery device (pump) 202 or the BG sensor 204. In other examples, the different functions of the AP application may be performed by one device, such as the management device 206, the wearable drug delivery device (pump) 202 or the BG sensor 204. In various examples, the drug delivery system 200 may include features of or may operate according to functionalities of a drug delivery system as described in U.S. Patent Application Nos. 15/359,187, filed November 22, 2016 and 16/570,125, filed September 13, 2019.

As described herein, the drug delivery system 200 or any component thereof, such as the wearable drug delivery device may be considered to provide AP functionality or to implement an AP application. Accordingly, references to the AP application (e.g., functionality, operations, or capabilities thereof) are made for convenience and may refer to and/or include operations and/or functionalities of the drug delivery system 200 or any constituent component thereof (e.g., the wearable drug delivery device 202 and/or the management device 206). The drug delivery system 200 - for example, as an insulin delivery system implementing an AP application - may be considered to be a drug delivery system or an AP application-based delivery system that uses sensor inputs (e.g., data collected by the BG sensor 204).

In an example, the drug delivery device 202 includes a communication device 264, which as described above may be a receiver, a transmitter, or a transceiver that operates according to one or more radio-frequency protocols, such as Bluetooth, Wi-Fi, a near-field communication standard, a cellular standard, that may enable the respective device to communicate with the cloud-based services 211. For example, outputs from the BG sensor 204 or the wearable drug delivery device (pump) 202 may be transmitted to the cloud-based services 211 for storage or processing via the transceivers of communication device 264. Similarly, wearable drug delivery device 202, management device 206 and BG sensor 204 may be operable to communicate with the cloud-based services 211 via the communication link 288.

In an example, the respective receiver or transceiver of each respective device 202, 206 or 207 may be operable to receive signals containing respective BG measurement values of the number of BG measurement values that may be transmitted by the BG sensor 204. The respective processor of each respective device 202, 206 or 207 may be operable to store each of the respective BG measurement values in a respective memory, such as 223, 263 or 273. The respective BG measurement values may be stored as data related to the AP algorithm, such as 229, 249, or 269. In a further example, the AP application operating on any of the management device 206, the smart accessory device 207, or BG sensor 204 may be operable to transmit, via a transceiver implemented by a respective communication device, 264, 274, 246, a control signal for receipt by a wearable drug delivery device. In the example, the control signal may indicate an amount of insulin to be expelled by the wearable drug delivery device 202.

In an example, one or more of the devices 202, 204, or 206 may be operable to communicate via a wired communication links 277, 278 and 279, respectively. The cloud-based services (not shown) may utilize servers and data storage (not shown). A communication link that couples the drug delivery system 200 to the cloud-based services may be a cellular link, a Wi-Fi link, a Bluetooth link, or a combination thereof, that is established between the respective devices 202, 204, or 206 of system 200. For example, the data storage (not shown) provided by the cloud-based services may store anonymized data, such as user weight, BG measurements, age, meal carbohydrate information, or the like. In addition, the cloud-based services 211 may process the anonymized data from multiple users to provide generalized information related to the various parameters used by the AP application. For example, an age-based general target BG value related to activity levels or particular exercises or sports may be derived from the anonymized data, which may be helpful when a user selects an activity mode (or a hyperglycemia protect mode, or a hypoglycemia protect mode) or the system 200 automatically implements the activity mode (or the hyperglycemia protect, or the hypoglycemia protect modes). The cloud-based services may also provide processing services for the system 200, such as performing a process described with reference to later examples.

The wearable drug delivery device 202 may also include a user interface 227. The user interface 227 may include any mechanism for the user to input data to the drug delivery device 202, such as, for example, a button, a knob, a switch, a touch-screen display, or any other user interaction component. The user interface 227 may include any mechanism for the drug delivery device 202 to relay data to the user and may include, for example, a display, a touch-screen display, or any means for providing a visual, audible, or tactile (e.g., vibrational) output (e.g., as an alert). The user interface 227 may also include additional components not specifically shown in Figure 2 for sake brevity and explanation. For example, the user interface 227 may include one or more user input or output components for receiving inputs from or providing outputs to a user or a caregiver (e.g., a parent or nurse), a display that outputs a visible alert, a speaker that outputs an audible, or a vibration device that outputs tactile indicators to alert a user or a caregiver of a potential activity mode, a power supply (e.g., a battery), and the like. Inputs to the user interface 227 may, for example, be a via a fingerprint sensor, a tactile input sensor, a button, a touch screen display, a switch, or the like. In yet another alternative, the activity mode of operation may be requested through a management device 206 that is communicatively coupled to a controller 221 of the wearable drug delivery device 202. In general, a user may generate instructions that may be stored as user preferences in a memory, such as 223 or 263 that specify when the system 200 is to enter the activity mode of operation.

Various operational scenarios and examples of processes performed by the system 200 are described herein. For example, the system 200 may be operable to implement process examples related to an activity mode including a hyperglycemia protect mode and a hypoglycemia protect mode as described in more detail below.

In an example, the drug delivery device 202 may operate as an AP system (e.g., as a portion of the AP system 100) and/or may implement techniques or an algorithm via an AP application that controls and provides functionality related to substantially all aspects of an AP system or at least portions thereof. Accordingly, references herein to an AP system or AP algorithm may refer to techniques or algorithms implemented by an AP application executing on the drug delivery device 202 to provide the features and functionality of an AP system. The drug delivery device 202 may operate in an open-loop or closed-loop manner for providing a user with insulin.

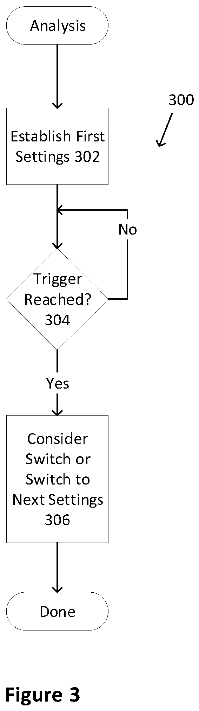

Additional features may be implemented as part of the AP application such as the activity mode, the hyperglycemia mode, the hypoglycemia mode, or the like. As the AP application, including the programming code for the activity mode, the hyperglycemia mode, and the hypoglycemia mode is executed, the AP application may adjust operations, such as detecting motion or movement of the wearable drug delivery device that is indicative of physical activity of the user. For example, motion and movement of the wearable drug delivery device 202 that induces motions characteristic of physical activity of the user (e.g., movements, such as jumping, dancing, running, weightlifting, cycling or the like) may be detected by the IMU 207. In addition, the IMU 207, as described with reference to Figure 3, may include a global positioning system that may detect a location of the wearable drug delivery device 202. Alternatively, or in addition, the wearable drug delivery device 202 may also utilize Wi-Fi location services to determine the location of the wearable drug delivery device 202. For example, the AP algorithm may learn from repeated interaction with the user who may input an indication at particular times that they are about to perform physical activity. Alternatively, or in addition, the wearable drug delivery device 202 may upon detection of a particular location (e.g., gym, sports field, stadium, track, or the like) determine that the user is about to increase their physical activity.

As was discussed above, the AID system typically is preconfigured with a generic set of settings that are not customized to a user. The settings are chosen to be "safe," so as to minimize the risk of undesirable outcomes due to the user not managing glucose control well. As was also discussed above, it may be desirable for a user who shows good quality glucose control to have more aggressive or less aggressive settings. The exemplary embodiments provide that ability to automatically adjust (or recommend adjusting) the settings to a different degree of aggressiveness.

Figure 3 depicts a flowchart 300 of illustrative steps that may be performed in adjusting the settings to settings of a different level of aggressiveness or providing the ability to adjust the settings to a different level of aggressiveness. Initially, the first settings are established (302). The first settings may be the factory settings that are established by default. Alternatively, these settings may have been set by a medical professional or even chosen by the user of the AID system. These settings may be, for example, the control BG level target, the constraints on the control algorithm, the weights applied in the cost function, and other settings. At some point during operation of the AID system, a trigger is reached (304). Until the trigger is reached, the first settings remain in effect. The trigger causes the system to either consider adjusting the settings or to switch to the next settings of a different degree of aggressiveness.

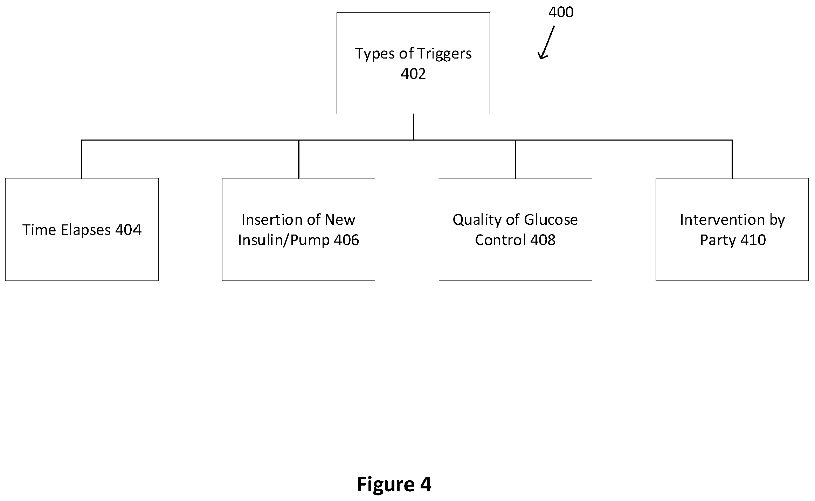

Figure 4 depicts a diagram 400 showing illustrative types of triggers 402. A first type of trigger is the elapsing of a time period 404. The AID system may be configured to initiate or consider initiating the switching to the second settings after an interval of time has elapsed. The interval may be a repeated, fixed size interval, such as after a week, several weeks or the like, or may be a variable sized interval, such as initially after a week, then after two weeks, etc. The interval also need not be repeatable but instead can be a singular instance.

The trigger may also be the insertion of more insulin, such as a new packet of insulin or a new reservoir of insulin, or the swapping in of a new pump 406. Some AID systems rely on disposable pumps that must be replaced regularly, such as after every three days. The trigger may also be based on the quality of the glucose control 408, such as a history of maintaining good quality glucose control for a period of time, or a history of maintaining poor glucose control for a period of time. The trigger may also be intervention by a party 410, such as the user, a medical professional, or a caretaker for the user, such as a parent.

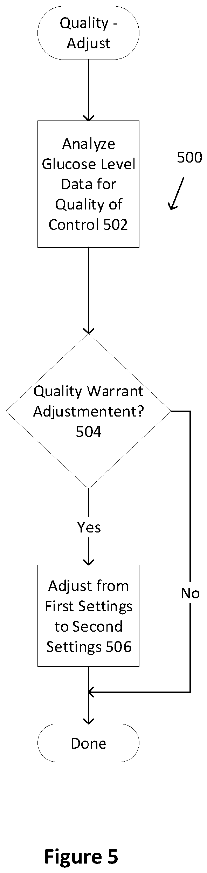

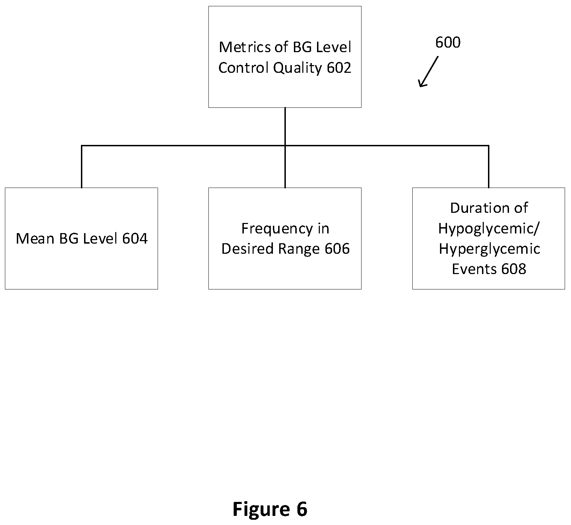

Figure 5 depicts a flowchart 500 of steps that may be performed when the quality of glucose control is examined to determine whether to change the settings. First, the BG level data for the user is analyzed to determine the quality of the glucose control (502). This may entail looking at metrics of BG level control quality 602, like those shown in the diagram 600 of Figure 6. These metrics are intended to be illustrative and not exhaustive. A first metric of BL level control quality 602 that may be examined in the mean glucose level 604. This is the mean of the BG level for the user over the period for which data is being examined. A mean that is not close to the hypoglycemic threshold or the hyperglycemic threshold is generally desirable. Another metric of BG level control quality 602 is the frequency that the BG level remains in a desired range (time in range or TIR) 606. Remaining in the desired range for a high frequency is desirable. For example, the BG level of a user may remain in a desired range 95% of the time. In general, a larger frequency is desirable. A third metric of BG level control quality 602 is the number and duration of hypoglycemic events or hyperglycemic events 608. Short duration hypoglycemic events or hyperglycemic events generally are desirable. This third metric may involve a sum of the number of hypo- or hyper-glycemic events multiplied by the duration of each event to obtain a number that may be compared to a threshold.

The quality of BG level control is assessed differently based on the user's ingestion of meals with carbohydrate content. Regardless of the safety and effectiveness of the system, the user may experience elevated glucose concentrations following ingestion of carbohydrates. As these periods of elevated BG concentrations may not be directly reflective of the quality of glucose control, one exemplary embodiment may ignore elevated glucose following carbohydrate ingestions for its BG level control quality assessment. In this instance, the determination of carbohydrate ingestion may be executed either through the presence of a meal carbohydrate entry, or by the presence of a manual insulin delivery request that exceeds the user's current insulin needs to correct to their glucose target. This insulin need can be calculated by dividing the difference between the user's current BG and the control target with the user's correction factor, a clinical parameter that defines the amount of glucose deviation that is compensated per 1 U of insulin delivery. The correction factor may either be entered by the user or calculated based on heuristics with TDI, such as 1800/TDI, where the 1800 value can vary between 1600 to 2400. Once the carbohydrate ingestion is detected, any glucose control outcomes within 90 minutes to 5 hours following the ingestion point are not included in the overall assessment of BG level control quality.

These metrics of BG level control quality may be used separately or in combination to determine the quality of BG level control (502). For example, the mean BG level may be used alone to determine BG level control quality. Suppose for example that the range of desirable glucose levels is 110 mg/dL to 150 mg/dL. In an example instance, the quality of the BG level control by the user may be determined as being high if the mean falls within the range. Alternatively, the process may look at both mean BG level 604 and the frequency in the desired range 606. In this case, the quality of BG level control is deemed to be high if the mean glucose level 604 is in the desired range and the BG level remained in the desired range 95% of the time. It will be appreciated that other combinations of these metrics 602 may be applied to determine quality of glucose control by the user for a period. Moreover, other metrics may be used as well as, or in place of, the illustrative metrics 604, 606 and 608.

Based on the metrics of BG level control quality a determination is made whether the determined quality warrants an adjustment in the settings of the AID system (504). In some instances, a quality score may be generated and compared to a threshold to determine if the quality is in range for an adjustment. In other instances, conditions may be checked as described above to see if the quality warrants adjustment. More generally, a determination may be made whether the user controlled the BG levels well or poorly and thus whether changes to the settings are warranted. If no adjustment is warranted, no adjustments are made to the settings, and the first settings remain in place. If an adjustment is warranted, the first settings are adjusted to the second settings (506). The second settings are either more aggressive or less aggressive than the first settings depending on the aim of the AID system in making the adjustments.

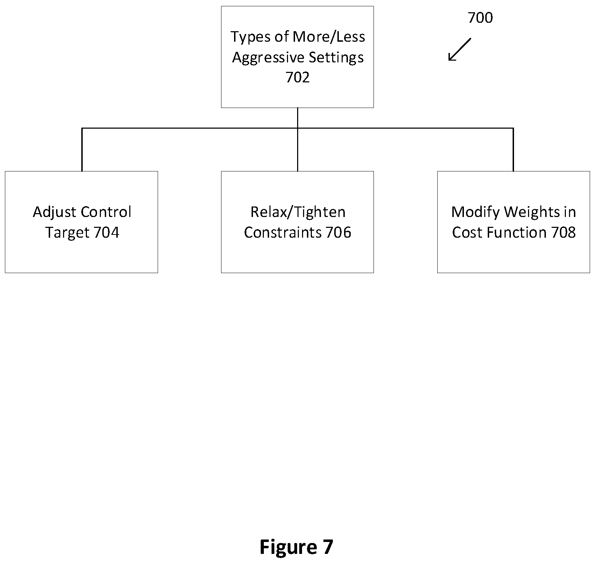

Figure 7 depicts a diagram 700 showing examples of types 702 of more aggressive settings or less aggressive settings that may result from the adjustment from the first settings to the second settings. A first type of more aggressive or less aggressive setting may be adjusting the control target to a new value 704 in a closed loop control system for the AID system. For example, with more aggressive settings a control target for the BG level may be lowered to be closer to a hypoglycemic threshold or may be increased to be closer to a hyperglycemic threshold. With less aggressive settings, the control target may be moved to be more centrally positioned within a desired range of BG levels for the user.

A second type of more aggressive or less aggressive settings are settings that relax constraints or tighten constraints 706 of the AID system. For example, the AID may have a constraint that limits the size of a basal insulin dosage. Increasing that dosage constraint is a more aggressive setting and decreasing that dosage constraint is a less aggressive setting.

A third type of more aggressive or less aggressive settings are settings that change the weights in the cost function 708. This may cause the cost function to more heavily weigh or less heavily weigh certain costs. For instance, the cost associated with each insulin delivery may be increased or decreased.

In the example of Figure 5, the quality of the BG level control causes the settings to be changed or maintained. As was mentioned above, the quality of the BG level control need not cause the change to second settings but may instead only enable the second settings to be enabled and available for selection.

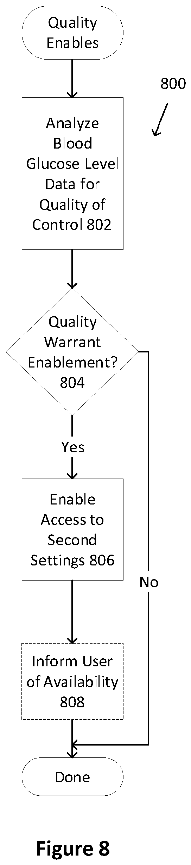

Figure 8 depicts a flowchart 800 of illustrative steps that may be performed in such an instance. Initially, the BG level data is analyzed for quality of control (802) as was described above (see 502). Based on the quality of BG level control, a decision is made whether the second settings are enabled or not (804). This decision may entail looking at the previously described metrics of quality and based on the metrics, making the decision to enable the second settings or not. If the decision is that the additional settings are warranted, access to the second settings is enabled (806). The user may then select the second settings or remain with the first settings. Optionally, the user may be informed of the enablement of the second settings (808). This may involve sending the user a message or notice. As an alternative, the second settings may become visible to the user for selection, whereas they previously may have been greyed out or not visible to the user. If the quality does not warrant enablement (see 804), no enablement is performed.

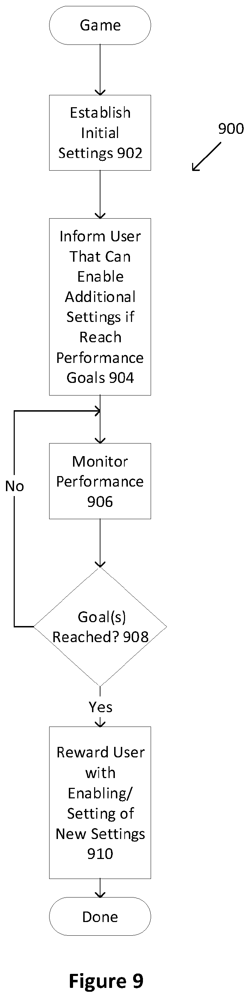

The access to the second setting may be gained as part of a game where the user strives to provide high quality BG level control to gain access to the second settings. Figure 9 provides a flowchart 900 of steps that may be performed in such an instance. The initial settings are established (902). The user is informed that additional settings may be enabled if performance goals are reached (904). The user may be informed by a message, a notice or via a user interface. The performance goals may involve achieving particular metric values for metrics of BG level control. The performance of the user may be monitored by the AID system

(906). When one or more goals are reached (908), the user is rewarded with enablement of one or more new settings (910). Suppose, for example, the user maintains their BG level within the desired range 95% of the time for a week. The user may then be rewarded with an expansion of the range of available control BG level targets that may be selected. As other performance goals are reached, an additional setting may be enabled or alternatively multiple settings may be enabled concurrently. In some alternative embodiments, the settings are not only enabled but are actually set automatically in response to achieving the performance goal(s) (910).

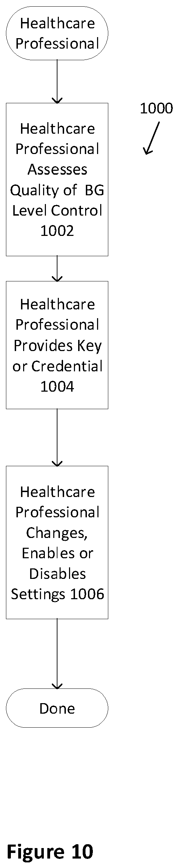

As has been discussed above, the AID system of the exemplary embodiments may envision participation by a healthcare professional. Figure 10 depicts a flowchart 100 of steps that may be performed regarding a healthcare professional. A healthcare professional, like a physician of a user, or a caretaker, may assess the quality of BG level control that has been exhibited by the user (1002). Based on the assessment, the healthcare professional determines that an adjustment in the settings of the AID system should be made or enabled. To that end, the healthcare professional provides a key or other secure evidence of credentials to the AID system, or otherwise enables the modified settings (1004). Selected settings of the AID system may then be enabled, set or even disabled (1006) by the healthcare professional.

The exemplary embodiments enable a user to have more aggressive settings after demonstrating good quality blood glucose level control. This allows the settings to be more quickly customized to a user that demonstrates good quality blood glucose level control than with conventional systems. As the user has demonstrated good quality blood glucose level control, there is less need to constrain the settings and provide a high margin of safety.

The access to the more aggressive settings may be gamified so that good quality blood glucose level control by a user results in access to more aggressive settings. When the access is gamified, the user may be informed of blood glucose level control performance goals and that reaching the goals will result in access to the more aggressive settings. This provides an incentive for the user to practice good quality blood glucose level control.

In some exemplary embodiments, the user may be transitioned to less aggressive settings based on other factors, such as a history of poor quality blood glucose level control, age, health, cognitive state, etc. This may provide a higher margin of safety for the user and may be more convenient for some users.

While exemplary embodiments have been described herein, it should be appreciated that various changes in form and detail may be made within the scope of the appended claims.

## Claims

1. A non-transitory computer-readable storage medium storing instruction that when executed by a processor of an automated insulin delivery (AID) system (200) performs the following method (300, 500, 800) steps:
establishing first settings for a control approach of the AID system, wherein the first settings include at least one of:
a control target blood glucose level range for the user and/or a cost function used in determining how much insulin to deliver to the user;
analyzing (502, 802) with the processor data regarding a user of the AID system to assess quality of blood glucose level control by the user with the AID system, wherein outcomes within 90 minutes to 5 hours following detection of carbohydrate ingestion are excluded from the analysis, wherein analyzing the data regarding the user comprises analyzing at least one of a mean blood glucose level of the user, a measure of how often the blood glucose level of the user has remained within a range, or a duration of hypoglycemic or hyperglycemic events for the user;
determining (304, 504, 804) whether the assessed quality warrants a change in settings;
where the assessed quality warrants a change in settings, with the processor:
automatically adjusting (306, 506) first settings of the AID system to second settings that are a different degree of aggressiveness than the first settings, where the degree of aggressiveness reflects an amount of hypoglycemic risk or hyperglycemic risk potential that is posed by the settings, wherein the second settings result in at least one of changing the control target blood glucose level range for the user, or modifying weights of the cost function; or
providing (806, 808) a party with an ability to adjust from the first settings to the second settings; and
where the assessed quality does not warrant a change in settings, with the processor:
keeping the first settings.

2. The non-transitory computer-readable storage medium according to claim 1, wherein the analyzing (502, 802) the data regarding the user comprises analyzing multiple ones of the mean blood glucose level of the user, the measure of how often the blood glucose level of the user has remained within a range or a duration of hypoglycemic or hyperglycemic events for the user.

3. The non-transitory computer-readable storage medium according to one of the preceding claims, wherein the data regarding the user is for a fixed time period or a time period between insertions of a new supply of insulin for the AID system (200).

4. The non-transitory computer-readable storage medium according to one of the preceding claims, wherein the automatically adjusting (306, 506) to the second settings is permanent in that the settings cannot be changed again.

5. The non-transitory computer-readable storage medium according to one of the preceding claims that when executed by the processor of an automated insulin delivery system (200) performs the following additional method (300, 500, 800) step: analyzing data regarding the user with the processor for a time since the second settings have been set to assess the quality of blood glucose level control by the user with the AID system (200) for the time.

6. The non-transitory computer-readable storage medium according to claim 5 that when executed by the processor of an automated insulin delivery system (200) performs the following additional method (300, 500, 800) step:
disabling the second settings with the processor responsive to the assessing the quality of blood glucose level control by the user for the time since the second settings have been set.

7. The non-transitory computer-readable storage medium according to claim 6, wherein responsive to the assessing of the quality of the blood glucose level control by the user with the AID system (200) for the time:
with the processor, automatically adjusting the settings to third settings that differ from the first settings and the second settings, or
with the processor, providing the party with an ability to adjust the settings from the second settings to the third settings.

8. The non-transitory computer-readable storage medium according to one of the preceding claims that when executed by the processor of an automated insulin delivery system (200) performs the following additional method (300, 500, 800) step: notifying the user of a possibility of the second settings if the user demonstrates good glucose control.

9. The non-transitory computer-readable storage medium according to one of the preceding claims, wherein the second settings pose a greater potential degree of hypoglycemia risk.

10. The non-transitory computer-readable storage medium according to one of the preceding claims that when executed by the processor of an automated insulin delivery system (200) performs the following additional method (300, 500, 800) steps:
receiving a request from the user to revert back to the first settings for the AID system (200);
in response to the request, with the processor, reverting to the first settings for the AID system.

11. The non-transitory computer-readable storage medium according to one of the preceding claims that when executed by the processor of an automated insulin delivery system (200) performs the following additional method (300, 500, 800) step:
before the analyzing (502, 802), the second settings are not visible to the user or are visibly indicated as being unavailable to the user.

12. An automated insulin delivery system (200) having a processor, and **characterized by** a non-transitory computer-readable storage medium according to one of the preceding claims.

## Patentansprüche

1. Nicht-transitorisches computerlesbares Datenspeichermedium, das Anweisungen speichert, die, wenn sie von einem Prozessor eines automatisierten Insulinabgabesystems (AID-System) (200) ausgeführt werden, die folgenden Schritte eines Verfahrens (300, 500, 800) durchführen:
Einrichten erster Einstellungen für einen Regelungsansatz des AID-Systems, wobei die ersten Einstellungen wenigstens eines hiervon beinhalten:
einen Steuerziel-Blutzuckerspiegelbereich für den Benutzer und/oder eine Kostenfunktion, die verwendet werden, um zu bestimmen, wie viel Insulin an den Benutzer abgegeben werden soll;
Analysieren (502, 802), mit dem Prozessor, von Daten zu einem Benutzer des AID-Systems, um die Qualität einer Blutzuckerspiegelsteuerung durch den Benutzer mit dem AID-System zu beurteilen, wobei Resultate innerhalb von 90 Minuten bis 5 Stunden nach Detektion einer Kohlenhydrateinnahme von der Analyse ausgeschlossen werden, wobei das Analysieren der benutzerbezogenen Daten das Analysieren von wenigstens einem von einem mittleren Blutzuckerspiegel des Benutzers, eines Maßes, wie oft der Blutzuckerspiegel des Benutzers innerhalb eines Bereichs geblieben ist, oder einer Dauer von hypoglykämischen oder hyperglykämischen Ereignissen für den Benutzer umfasst;
Bestimmen (304, 504, 804), ob die beurteilte Qualität eine Änderung von Einstellungen rechtfertigt;
wenn die beurteilte Qualität eine Änderung von Einstellungen rechtfertigt, mit dem Prozessor:
automatisches Anpassen (306, 506) erster Einstellungen des AID-Systems an zweite Einstellungen, die einen anderen Aggressivitätsgrad darstellen als die ersten Einstellungen, wobei der Aggressivitätsgrad die Menge eines hypoglykämischen oder hyperglykämischen Risikopotenzials widerspiegelt, das von den Einstellungen ausgeht, wobei die zweiten Einstellungen wenigstens eines von dem Ändern des Steuerziel-Blutzuckerspiegelbereichs für den Benutzer oder dem Modifizieren von Gewichtungen der Kostenfunktion zur Folge haben; oder Bereitstellen (806, 808) einer Möglichkeit für eine Partei, eine Anpassung von den ersten Einstellungen zu den zweiten Einstellungen vorzunehmen; und
wenn die beurteilte Qualität keine Änderung von Einstellungen rechtfertigt, mit dem Prozessor:
Beibehalten der ersten Einstellungen.

2. Nicht-transitorisches computerlesbares Datenspeichermedium gemäß Anspruch 1, wobei das Analysieren (502, 802) der benutzerbezogenen Daten das Analysieren mehrerer mittlerer Blutzuckerspiegel des Benutzers umfasst, das Maß dafür, wie oft der Blutzuckerspiegel des Benutzers innerhalb eines Bereichs geblieben ist, oder einer Dauer von hypoglykämischen oder hyperglykämischen Ereignissen.

3. Nicht-transitorisches computerlesbares Datenspeichermedium gemäß einem der vorstehenden Ansprüche, wobei die benutzerbezogenen Daten für einen festen Zeitraum oder einen Zeitraum zwischen Einsetzungen eines neuen Insulinvorrats für das AID-System (200) gelten.

4. Nicht-transitorisches computerlesbares Datenspeichermedium gemäß einem der vorstehenden Ansprüche, wobei das automatische Anpassen (306, 506) an die zweiten Einstellungen dahingehend permanent ist, dass die Einstellungen nicht wieder geändert werden können.

5. Nicht-transitorisches computerlesbares Datenspeichermedium gemäß einem der vorstehenden Ansprüche, das bei Ausführung durch den Prozessor eines automatisierten Insulinabgabesystems (200) den folgenden zusätzlichen Schritt eines Verfahrens (300, 500, 800) durchführt:
Analysieren von benutzerbezogenen Daten mit dem Prozessor für eine Zeit, seit der die zweiten Einstellungen vorgenommen worden sind, um die Qualität einer Blutzuckerspiegelsteuerung durch den Benutzer mit dem AID-System (200) für die Zeit zu beurteilen.

6. Nicht-transitorisches computerlesbares Datenspeichermedium gemäß Anspruch 5, das bei Ausführung durch den Prozessor eines automatisierten Insulinabgabesystems (200) den folgenden zusätzlichen Schritt eines Verfahrens (300, 500, 800) durchführt:
Deaktivieren der zweiten Einstellungen mit dem Prozessor in Reaktion auf das Beurteilen der Qualität der Blutzuckerspiegelsteuerung durch den Benutzer für die Zeit, seit der die zweiten Einstellungen eingestellt worden sind.

7. Nicht-transitorisches computerlesbares Datenspeichermedium gemäß Anspruch 6, wobei in Reaktion auf das Beurteilen der Qualität der Blutzuckerspiegelsteuerung durch den Benutzer mit dem AID-System (200) für die Zeit:
mit dem Prozessor, die Einstellungen automatisch an dritte Einstellungen angepasst werden, die sich von den ersten Einstellungen und den zweiten Einstellungen unterscheiden, oder
mit dem Prozessor, der Partei eine Möglichkeit geboten wird, die Einstellungen von den zweiten Einstellungen an die dritten Einstellungen anzupassen.

8. Nicht-transitorisches computerlesbares Datenspeichermedium gemäß einem der vorstehenden Ansprüche, das bei Ausführung durch den Prozessor eines automatisierten Insulinabgabesystems (200) den folgenden zusätzlichen Schritt eines Verfahrens (300, 500, 800) durchführt:
Benachrichtigen des Benutzers über eine Möglichkeit der zweiten Einstellungen, falls der Benutzer eine gute Glukosesteuerung demonstriert.

9. Nicht-transitorisches computerlesbares Datenspeichermedium gemäß einem der vorstehenden Ansprüche, wobei die zweiten Einstellungen ein höheres potenzielles Ausmaß eines Hypoglykämierisikos darstellen.

10. Nicht-transitorisches computerlesbares Datenspeichermedium gemäß einem der vorstehenden Ansprüche, das bei Ausführung durch den Prozessor eines automatisierten Insulinabgabesystems (200) die folgenden zusätzlichen Schritte eines Verfahrens (300, 500, 800) durchführt:
Empfangen einer Anforderung von dem Benutzer, zu den ersten Einstellungen für das AID-System (200) zurückzukehren;
in Reaktion auf die Anforderung, mit dem Prozessor, Zurückkehren zu den ersten Einstellungen des AID-Systems.

11. Nicht-transitorisches computerlesbares Datenspeichermedium gemäß einem der vorstehenden Ansprüche, das bei Ausführung durch den Prozessor eines automatisierten Insulinabgabesystems (200) den folgenden zusätzlichen Schritt eines Verfahrens (300, 500, 800) durchführt:
vor dem Analysieren (502, 802) sind die zweiten Einstellungen für den Benutzer nicht sichtbar oder werden sichtbar als für den Benutzer nicht verfügbar angezeigt.

12. Automatisiertes Insulinabgabesystem (200), das einen Prozessor aufweist und durch ein nicht-transitorisches computerlesbares Datenspeichermedium gemäß einem der vorstehenden Ansprüche gekennzeichnet ist.

## Revendications

1. Support de stockage lisible par ordinateur non transitoire stockant des instructions qui, lorsqu'elles sont exécutées par un processeur d'un système automatisé d'administration d'insuline (AID) (200), réalisent les étapes de procédé suivantes (300, 500, 800) :
l'établissement de premiers réglages pour une approche de commande du système AID, les premiers réglages comprenant au moins l'un des éléments suivants :
une plage de niveau de glycémie cible de commande pour l'utilisateur et/ou une fonction de coût utilisée pour déterminer la quantité d'insuline à administrer à l'utilisateur ;
l'analyse (502, 802) avec le processeur de données concernant un utilisateur du système AID pour évaluer la qualité de commande du niveau de glycémie par l'utilisateur avec le système AID, les résultats dans les 90 minutes à 5 heures suivant la détection de l'ingestion de glucides étant exclus de l'analyse, l'analyse des données concernant l'utilisateur comprenant l'analyse d'au moins un niveau de glycémie moyen de l'utilisateur, une mesure de la fréquence à laquelle le niveau de glycémie de l'utilisateur est resté dans une plage, ou une durée d'événements hypoglycémiques ou hyperglycémiques pour l'utilisateur ;
la détermination (304, 504, 804) si la qualité évaluée justifie une modification des réglages ;
lorsque la qualité évaluée justifie une modification des réglages, avec le processeur :
le réglage automatique (306, 506) des premiers réglages du système AID à des deuxièmes réglages qui sont d'un degré d'agressivité différent des premiers réglages, le degré d'agressivité reflétant une quantité de risque hypoglycémique ou de potentiel de risque hyperglycémique qui est posé par les réglages, les deuxièmes réglages ayant pour résultat au moins un changement de la plage de niveau de glycémie cible de commande pour l'utilisateur, ou une modification des pondérations de la fonction de coût ; ou la fourniture (806, 808) à une partie de la capacité d'ajuster des premiers réglages aux deuxièmes réglages ; et
lorsque la qualité évaluée ne justifie pas un une modification des réglages, avec le processeur :
la conservation des premiers réglages.

2. Support de stockage lisible par ordinateur non transitoire selon la revendication 1, l'analyse (502, 802) des données concernant l'utilisateur comprenant l'analyse de plusieurs niveaux de glycémie moyens de l'utilisateur, la mesure de la fréquence à laquelle le niveau de glycémie de l'utilisateur est resté dans une plage ou une durée d'événements hypoglycémiques ou hyperglycémiques pour l'utilisateur.

3. Support de stockage lisible par ordinateur non transitoire selon l'une des revendications précédentes, les données concernant l'utilisateur concernant une période de temps fixe ou une période de temps entre les insertions d'une nouvelle alimentation en insuline pour le système AID (200).

4. Support de stockage lisible par ordinateur non transitoire selon l'une des revendications précédentes, l'ajustement automatique (306, 506) aux deuxièmes réglages étant permanent en ce que les réglages ne peuvent pas être modifiés à nouveau.

5. Support de stockage lisible par ordinateur non transitoire selon l'une des revendications précédentes, qui, lorsqu'il est exécuté par le processeur d'un système automatisé d'administration d'insuline (200), réalise l'étape de procédé supplémentaire suivante (300, 500, 800) :
l'analyse de données concernant l'utilisateur à l'aide du processeur pendant un certain temps depuis que les deuxièmes réglages ont été réglés afin d'évaluer la qualité de commande du niveau de glycémie par l'utilisateur à l'aide du système AID (200) pendant le temps.

6. Support de stockage lisible par ordinateur non transitoire selon la revendication 5, qui, lorsqu'il est exécuté par le processeur d'un système automatisé d'administration d'insuline (200), réalise l'étape de procédé supplémentaire suivante (300, 500, 800) :
la désactivation des deuxièmes réglages avec le processeur en réponse à l'évaluation de la qualité de commande du niveau de glycémie par l'utilisateur pendant le temps écoulé depuis que les deuxièmes réglages ont été réglés.

7. Support de stockage lisible par ordinateur non transitoire selon la revendication 6, en réponse à l'évaluation de la qualité de la commande du niveau de glycémie par l'utilisateur avec le système AID (200) pendant le temps :
avec le processeur, l'ajustement automatique des réglages sur des troisièmes réglages qui diffèrent des premiers réglages et des deuxièmes réglages, ou
avec le processeur, la fourniture à la partie de la possibilité d'ajuster les réglages des deuxièmes réglages au troisièmes réglages.

8. Support de stockage lisible par ordinateur non transitoire selon l'une des revendications précédentes, qui, lorsqu'il est exécuté par le processeur d'un système automatisé d'administration d'insuline (200), réalise l'étape de procédé supplémentaire suivante (300, 500, 800) :
la notification à l'utilisateur de la possibilité d'utiliser les deuxièmes réglages si l'utilisateur démontre une bonne commande de glycémie.

9. Support de stockage lisible par ordinateur non transitoire selon l'une des revendications précédentes, les deuxièmes réglages présentant un degré potentiel plus élevé de risque d'hypoglycémie.

10. Support de stockage lisible par ordinateur non transitoire selon l'une des revendications précédentes, qui, lorsqu'il est exécuté par le processeur d'un système automatisé d'administration d'insuline (200), réalise les étapes supplémentaires suivantes du procédé (300, 500, 800) :
la réception d'une demande de l'utilisateur de revenir aux premiers réglages pour le système AID (200) ;
en réponse à la demande, le retour par le processeur aux premiers réglages du système AID.

11. Support de stockage lisible par ordinateur non transitoire selon l'une des revendications précédentes, qui, lorsqu'il est exécuté par le processeur d'un système automatisé d'administration d'insuline (200), réalise l'étape de procédé supplémentaire suivante (300, 500, 800) :
avant l'analyse (502, 802), les deuxièmes réglages n'étant pas visibles pour l'utilisateur ou étant visuellement indiqués comme étant indisponibles pour l'utilisateur.

12. Système automatisé d'administration d'insuline (200) ayant un processeur, et **caractérisé par** un support de stockage lisible par ordinateur non transitoire selon l'une des revendications précédentes.
